(19) Europäisches Patentamt
European Patent Office
Office européen des brevets

(11) Veröffentlichungsnummer: **0 102 506**
**B1**

(12) # EUROPÄISCHE PATENTSCHRIFT

(45) Veröffentlichungstag der Patentschrift: **25.02.87**

(51) Int. Cl.⁴: **C 07 D 493/04**, C 08 G 18/32, C 08 G 18/14

(21) Anmeldenummer: **83107356.4**

(22) Anmeldetag: **27.07.83**

(54) **Verfahren zur Herstellung von flüssigen Dianhydrohexitol-Gemischen aus Hexitolen, und ihre Verwendung zur Herstellung von Polymeren.**

(30) Priorität: **06.08.82 DE 3229412**

(43) Veröffentlichungstag der Anmeldung: **14.03.84 Patentblatt 84/11**

(45) Bekanntmachung des Hinweises auf die Patenterteilung: **25.02.87 Patentblatt 87/9**

(84) Benannte Vertragsstaaten: **BE DE FR GB IT NL**

(56) Entgegenhaltungen:
**EP-A-0 061 055**
**DE-A-2 513 817**
**DE-A-3 002 762**
**DE-A-3 028 873**
**DE-A-3 109 532**

**CHEMICAL ABSTRACTS, vol. 76, No. 15, 10. April 1972, Columbus, Ohio, USA FRASER-REID BERT, HICKS, DAVID R. "Acid versus base hydrolysis of a disulfonylated hexitol. 1,4:3,6--Dianyhdro-D-iditol (D-isoidide) versus 2,3:4,5-dianhydro-D-iditol." Seite 441, Spalte 1, Abstract-no. 86 055a**
**BEILSTEINS HANDBUCH DER ORGANISCHEN 4. Auflage, SPRINGER-VERLAG, Berlin-Heidelberg-New York 1977, E III, IV, Band 19, Seite 989**
**BEILSTEINS HANDBUCH DER ORGANISCHEN CHEMIE, 4. Auflage, SPRINGER-VERLAG, Berlin-Göttingen-Heidelberg, 1958, E III, Band 1, Seite 2387**

(73) Patentinhaber: **BAYER AG, Konzernverwaltung RP Patentabteilung, D-5090 Leverkusen 1 Bayerwerk (DE)**

(72) Erfinder: **Salzburg, Herbert, Dr., Hahnenweg 3, D-5000 Köln 80 (DE)**
Erfinder: **Hajek, Manfred, Dr., Hahnenweg 1, D-5000 Köln 80 (DE)**
Erfinder: **Meyborg, Holger, Dr., Bergisch-Gladbacher- Strasse 1, D-5068 Odenthal (DE)**

(56) Entgegenhaltungen: (Fortsetzung)
**BEILSTEINS HANDBUCH DER ORGANISCHEN CHEMIE, 4. Auflage, VERLAG VON JULIUS SPRINGER, 1941, E II, Band 1, Seite 611**
**J. Ann. Chem. Soc. 68 (1946), 939-941**
**J. Ann. Chem. Soc. 67 (1945), S. 1865**
**J. Org. Chem. 29 (1964) S. 2979-2982**

EP 0 102 506 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Es wird ein Verfahren zur Herstellung von flüssigen 1,4-3,6-Dianhydro-hexitol-Isomerengemischen aus Hexitolen durch Wasserabspaltung und Isomerisierung auf der Diacylhexitolstufe beschrieben, in dem man mit organischen Carbonsäuren, Carbonsäureanhydriden und/oder -halogeniden und Kohlensäureesterderivaten gebildete Acylierungsprodukte der Hexitole (im wesentlichen Diacylhexitole) in Gegenwart von als saure Dehydratisierungsreagentien wirkenden Protonen oder Lewis-Säuren in flüssiger oder heterogener Form bei Temperaturen von 170 bis 240°C unter gleichzeitiger Wasserabspaltung isomerisiert und das gebildete, (di)acylierte Dianhydrohexitolgemisch durch Verseifung oder Umesterung in das Dianhydrohexitol-Isomerengemisch überführt. Das Verfahren kann mit oder ohne Isolierung der Zwischenprodukte durchgeführt werden. Erfindungsgegenstand ist auch die Verwendung derartiger Isomerengemische als Kettenverlängerungsmittel bei der Herstellung von Polyurethanen, vorzugsweise Polyurethanelastomeren. Ebenfalls werden Dianhydro-idit enthaltende Isomerengemische von Dianhydrohexitolen beansprucht, wie sie durch die Isomerisierungsreaktionen nach dem erfindungsgemäßen Verfahren zugänglich sind.

Die Verbindungsklasse der 1.4-3.6-Dianhydro-hexitole ist seit ca. 1880 bekannt und wird z.B. in folgenden Literaturstellen beschrieben:

1. Pauconier, Bull Soc. Chem. (1884) 41, 119;
2. L.F. Wiggins, J. Chem. Soc. 1945, 4;
3. Haworth, Heath, Wiggins, J.Chem.Soc. 1944, 155;
4. R. Montgomery, L.F. Wiggins, J.Chem.Soc. 1947, 433;
5. J.C. Goodwin, J.E. Hodge, D. Weisleder, Carbohydr. Res. 79, 133 (1980);
6. S. Soltzberg, - Advances Carbohydr. Chem., 25, 229 (1970);
7. Ropuszynski et al., Przem. Chem. 48, (11), 665-668 (1969);
8. Weisleder et al., Carbohydr. Res. 79, 133-141 (1969);
9. DE-OS 3 041 673 (EP-A 52 295);
10. DE-OS 3 041 626
11. L.F. Wiggins, Adv. Carbohydrate Chem. 5, 191 (1950).

Den Synthesen ist gemeinsam, daß alle, ausgehend von einem Hexitol, das analoge Dianhydrohexitol liefern, wobei die Ausbeuten beträchtlich variieren. Der grundlegende Nachteil dieser Synthesen ist oftmals in einer nicht befriedigenden Ausbeute, den harzartigen Rückständen der Reaktion und besonders für den technischen Einsatz in der hohen Kristallinität der Produkte begründet. Auf Grund der ausgeprägten Kristallisationstendenz der Dianhydrohexitole lassen sich durch einfaches Mischen verschiedener Isomeren, gegebenenfalls unter Aufschmelzen bei Raumtemperatur, keine flüssig bleibenden, nicht kristallsierenden Isomerenmischungen erhalten. Daher besteht die Forderung soweit die Anhydrohexitole, insbesondere auf dem Polyurethangebiet eingesetzt werde daß sie in einer nicht lösungsmittelhaltigen flüssigen Form ohne Verwendung substanzklassenfremder Beimengungen vorliegen, da eine flüssige Form verarbeitungstechnisch einfacher und problemloser handhabbar ist. Der nötige Aufschmelzvorgang und das Flüssighalten der Substanz stellen einen energetisch aufwendigen zusätzlichen Verfahrensschritt dar, der für die Verarbeitung unerwünscht ist.

In der Literatur ist über Isomerisierungsreaktionen wenig bekannt. Es wurde lediglich von L.W. Wright et al., J.Org.Chem. 29, 2979 (1964) eine Isomerisierung von Dianhydrohexitolen durch thermische Behandlung bei sehr hohen Temperaturen (222-240°C) im Autoklaven in Gegenwart von Hydrierungskatalysatoren und Wasserstoff unter hohem Druck (150 at) eine Isomerisierung erreicht. Ohne Gegenwart von Wasserstoff unter hohem Druck läßt sich eine solche Isomerisierung jedoch nicht erfolgreich durchführen.

In Beilsteins Handbuch der Organischen Chemie, 4. Auflage 19/2 (1977) Seite 989 wird aus D-Glucit beim Erhitzen mit Chlorwasserstoff in Essigsäure auf 140°C 1,4-3,6-Dianhydroglucit erhalten (Kristalle, Fp 61,9-64°C). Es wird jedoch nicht die Isomerisierung beschrieben, auch benutzt das Verfahren nicht die Hydrolysestufe.

In einer eigenen, nicht vorveröffentlichten Anmeldung (EP-A-61055) wird ein Verfahren beschrieben, in dem lediglich katalytische Mengen an Acylierungsmitteln eingesetzt werden und keine Isomerisierungsreaktion auf der Diacylstufe und keine Hydrolyse erfolgt. Entsprechend werden andere Verfahrensprodukte erhalten.

Dianhydrosorbit ist nach eigenen Versuchen ein geeigneter Baustein für das Isocyanat-Polyadditionsverfahren ebenso wie Dianhydromannit. Beide Substanzen sind bei Raumtemperatur Feststoffe, deren Kristallisation auch in Gemischen wie oben gesagt nicht verhindert werden kann.

Die Aufgabe der vorliegenden Erfindung war die Auffindung eines Verfahrens zur Synthese von flüssigen Dianhydrohexitolen. Überraschenderweise wurde nun in dem hier beschriebenen Verfahren gefunden, daß während der Dehydratisierung von Hexitolen mit den aus der Literatur bekannten sauren Katalysatoren, wie auch allgemein jeder Protonen- und/oder Lewis-Säure, auf der Stufe (di)acylierter Hexitole unter Anwendung erhöhter Temperaturen auch eine Isomerisierung eintritt, wodurch nach Hydrolyse Dianhydrohexitol-Isomerengemische erhalten werden. So wird z.B. aus Sorbit im wesentlichen eine Mischung von 1,4-3,6-Dianhydrosorbit, 1,4-3,6-Dianhydromannit und 1,4-3,6-Dianhydroidid erzeugt. Nebenprodukte entstehen nach den erfindungsgemäßen Verfahren in nur geringer Menge, insbesondere sind die Anteile aus Monoanhydroverbindungen nur gering.

In nicht vorhersehbarer Weise sind die durch die erfindungsgemäße Isomerisierungsreaktion hergestellten Isomerengemische flüssig und von außerordentlich geringer Kristallisationsneigung, während zusammengesetzte Mischungen aus den Einzelkomponenten Dianhydrosorbit und Dianhydromannit die starke Kristallisationsneigung auch nach dem Zusammenschmelzen beibehalten.

Nach dem erfindungsgemäßen Verfahren sind überraschend aus dem wohlfeilen Sorbit als Ausgangsmaterial flüssige Dianhydrohexitolgemische zugänglich, welche neben Dianhydrosorbit auch den (isoliert hergestellten teureren) Dianhydromannit und den (sonst außerordentlich schwer zugänglichen) Dianhydroidit liefern. Der Dianhydroidit liegt in den Isomerengemischen in Mengen von 0,5-12 Gew.-%, vorzugsweise 2-9 Gew.-% vor.

Gegenstand der Erfindung ist ein Verfahren zur Herstellung von 1,4-3,6-Dianhydrohexitolen durch Wasserabspaltung aus Hexitolen in Gegenwart von starken Säuren und Acylierungsmitteln bei Temperaturen ⩾ 130° C, dadurch gekennzeichnet, daß man

Sorbit und/oder Mannit, vorzugsweise in Abwesenheit von Wasser oder einem zusätzlichen Lösungsmittel, mit 1,5-2,5 Äquivalenten an unter den Reaktionsbedingungen acylierend wirkenden Substanzen aus der Reihe der Carbonsäuren oder ihrer Anhydride, Halogenide oder Kohlensäureesterderivate sowie Keten pro Mol Hexitol in die Diacylierungsprodukte überführt, evtl. überschüssiges Acylierungsmittel und evtl. gebildetes Wasser, HCl oder Carbonsäuren entfernt,

die Diacylierungsprodukte der Hexitole in Gegenwart von 0,001 bis 10 Mol-% löslichen Proton- oder Lewissäuren oder heterogenen, sauren Katalysatoren bei Temperaturen von 170-240° C unter Wasserabspaltung und Isomerisierung zu den im wesentlichen diacylierten Dianhydrohexitol-Isomerengemischen umsetzt

und das acylierte Dianhydrohexitol-Isomerengemisch, gegebenenfalls nach der Destillation, durch Verseifung oder Umesterung in ein flüssiges 1,4-3,6-Dianhydrohexi-tol-Isomerengemisch von geringer Kristallisationsneigung und mit einem Gehalt an 0,5-12 Gew.-% an 1,4-3,6-Dianhydroidit -Isomeren überführt.

Gegenstand der Erfindung sind auch 0,5 bis 12 Gew.-% 1,4-3,6-Dianhydro-idit enthaltende flüssige, geringe Kristallisationsneigung zeigende Isomerengemische von 1,4-3,6-Dianhydro-sorbit und -mannit, wie sie durch Isomerisierungsreaktionen aus Sorbit und/oder Mannit, besonders bevorzugt aus Sorbit nach dem beanspruchten verfahren zugänglich sind.

Gegenstand der Erfindung ist auch die Verwendung der nach dem erfindungsgemäßen Verfahren hergestellten Dianhydrohexitol-Isomerengemische vorstehend gegebener Kennzeichnung als Kettenverlängerungsmittel, gegebenenfalls in Abmischung mit üblichen Kettenverlängerungsmitteln bei der Herstellung von Polyurethanen, vorzugsweise Polyurethanelastomeren.

Als Acylierungsmittel werden im erfindungsgemäßen Herstellungsverfahren Carbonsäuren, ihre Anhydride, Halogenide, einschließlich Kohlensäureesterderivate und Keten eingesetzt.

Als Carbonsäuren werden Monocarbonsäuren der allgemeinen Formel R-COOH, wobei R ein Rest einer aliphatischen gesättigten oder ungesättigten, einer aromatischen oder araliphatischen Carbonsäure ist, eingesetzt, z.B. Ameisensäure, Essigsäure, Propionsäure, Buttersäure, Valeriansäure, Hexansäure, Benzoesäure, Phenylessigsäure, Chloressigsäure, Triflouressigsäure, p-Chlor-benzoesäure. Di- und Polycarbonsäuren sind auch verwendbar sind jedoch weniger geeignet.

Als Säurehalogenide kommen vorzugsweise Säurechloride oder Säurebromide der oben erwähnten Carbonsäuren in Betracht. Beispiele sind Acetylchlorid, Propionylchlorid, Hexanoylchlorid, Benzoylchlorid, Phenylacetylchlorid und Oxalylchlorid sowie Acetylbromid.

Als Säureanhydride kommen symmetrische oder unsymmetrische Carbonsäureanhydride in Betracht, z.B. Acetanhydrid, Propionsäureanhydrid, gemischte Anhydride aus z.B. Essigsäure und Propionsäure oder Chlorameisensäureestern und Carbonsäuren.

Bevorzugte Acylierungsmittel sind Essigsäure, Acetylchlorid, Keten und ganz besonders das Acetanhydrid.

Die Acylierungsmittel werden in einer Menge von 1,5 und 2,5 Äquivalenten Acylierungsmittel bevorzugt zwischen 1,9 und 2,2 Äquivalenten gehalten.

Die Acylierungsreaktion wird üblicherweise in den Acylierungsmitteln angepaßter Form durchgeführt, z.B.:

mit Essigsäure unter Veresterung bei hohen Temperaturen und Einsatz eines sauren Katalysators, vorzugsweise kontinuierlichem Wasser-Abdestillieren und (bei Überschuß) unter Abbruch bei Erreichen der Diacylstufe;

mit Acetylchlorid (unter Entweichen von HCl) und Einhalten in etwa der geforderten Stöchiometrie;

mit Acetanhydrid unter Veresterung beim Erwärmen und unter Einhaltung etwa der geforderten Stöchiometrie (Reaktion vorzugsweise ohne Katalysator: etwa 2 Mol Acetanhydrid; bzw. mit Säure-Katalysator unter Veresterung auch der gebildeten Essigsäure: etwa 1 Mol Acetanhydrid); mit Keten im wesentlichen unter Einhaltung der Stöchiometrie.

Die Diacylhexitol-Stufe kann isoliert werden, doch wird in der Praxis die Wasserabspaltungs/Isomerisierungs-Stufe direkt angeschlossen, indem man evtl. überschüssige Acylierungsmittel und evtl. noch vorhandene Reaktionsprodukte ($H_2O$, HCl oder Carbonsäuren) entfernt.

Zur Wasserabspaltung aus dem Diacylhexitol wird der saure Katalysator, gegebenenfalls kontinuierlich über längeren Zeitraum, zugegeben und auf 170 bis 240°C, vorzugsweise 175 bis 210°C, ansteigend erhitzt, wobei vorzugsweise das gebildete Wasser gleichzeitig abdestilliert wird. Der saure Katalysator wird in 0,001 bis 10 Mol-%, vorzugsweise in 0,1 bis 5 Mol-% zugeführt.

Je nach Ansatzgröße und Apparatur wird solange erhitzt, bis die Wasserabspaltung beendet ist, z.B. 1 bis 2 Stunden bei 180 bis 195°C.

Als Katalysatoren für die Wasserabspaltungs- und Isomerisierungsreaktionen der Diacylhexitole zu den Diacyldianhydrohexitol-Isomeren kommen neben den Mineralsäuren wie Salzsäuren bzw. Chlorwasserstoff, Schwefelsäure, Phosphorsäure etc. auch Lewis-Säuren wie Bortrifluorid, Antimonpentachlorid, Zinn-(IV)-chlorid, sowie saure Ionenaustauscher auf Basis von z.B. mit Diphenylbenzol vernetzten Polystyrolsulfonsäurekationen-Austauscherharzen oder saure Zeolithe bzw. saure Crack- oder Hydrocrack-Katalysatoren in Betracht.

Die gebildeten Anhydrohexitole können z.B. durch Hochvakuumdestillation in Eorm hochsiedender Öle isoliert werden oder werden aus der Wasserabspaltungs-/Isomerisierungs-Reaktionsstufe direkt weiter umgesetzt, d.h. die Diacylgruppe wird abgespalten unter Bildung der Dianhydrohexitol-Gemische. Die Abspaltung der Acylgruppen erfolgt üblicherweise mit wäßrigen Basen, z.B. wäßrigen Alkalilaugen unter Hydrolyse oder durch (katalytische) Umesterung, z.B. in Alkoholen, mit einer katalytischen Menge eines Alkoholates.

Das entstandene Dianhydrohexitol-Isomerengemisch wird (gegebenenfalls nach Neutralisation) durch fraktionierende Destillation im Vakuum gewonnen oder kann durch Extraktion der wäßrigen Reaktionsmischung mit z.B. Essigsäurealkylestern oder Diethylketon erhalten werden.

Bevorzugtes Ausgangs-Hexitol ist Sorbit.

Die so gewonnenen 1,4-3,6-Dianhydrohexitol-Isomeren-gemische sind wertvolle Bausteine für das Diisocyanat-Polyadditionsverfahren, brauchbar als Starter für Polyether, z.B. durch Addition von Ethylenoxid und/oder Tropylenoxid, als Bausteine für Polyester, als Ausgangspolyol für Polycarbonate, als Härter für Epoxidharze sowie als Zwischenprodukte für Pharmaka.

Ganz besonders geeignet sind die flüssigen Dianhydrohexitolgemische als Kettenverlängerungsmittel für Polyurethanelastomere.

Erfindungsgemäß finden die so hergestellten Dianhydrohexitol-Isomerengemische Verwendung in Verfahren zur Herstellung von homogenen und zellförmigen Polyurethankunststoffen durch Umsetzung von

a) organischen Polyisocyanaten mit

b) mindestens 2 Zerewitinoff-aktive Wasserstoffatome aufweisenden Verbindungen der Molmasse 400 bis 10 000;

c) Kettenverlängerungsmitteln

d) gegebenenfalls weiteren mindestens 2 Zerewitinoff-aktive Wasserstoffatome aufweisenden kurzkettigen Verbindungen der Molmasse 32 bis 399,

e) sowie gegebenenfalls Katalysatoren, Treibmitteln und weiteren an sich bekannten Zusatzststoffen,

dadurch gekennzeichnet, daß man als Kettenverlängerer c) die nach den beanspruchte Verfahren hergestellten, flüssigen Isomerengemische der 1 4-3 6-Dianhydrohexitole entsprechend Ansprüchen 7 oder 8 gegebenenfalls in Abmischung mit niedermolekularen Verbindungen d) der Molmasse 62-339, einsetzt.

Die neuen Kettenverlängerungsmittel ergeben hochwertige Elastomere und Schaumstoffe. Bei Abmischung mit z.B. Butandiol-1,4 sind die Dianhydrohexitol-Isomerengemische in flüssiger, niederviskoser Form ohne Tendenz zum Auskristallisieren als Kettenverlängerungsmittel verwendbar.

Geeignete Polyisocyanate a) (vorzugsweise Diisocyanate) werden in der DE-A-2 920 501, Seite 13, Zeile 13 bis Seite 16 beschrieben. Bevorzugte Polyisocyanate sind: Die technisch leicht zugänglichen Polyisocyanate, z.B. das 2,4- und 2,6-Toluylendiisocyanat sowie beliebige Gemische dieser Isomeren (TDI), Polyphenylpolymethylen-polyisocyanate, wie sie durch Anilin-Formaldehyd-Kondensation und anschließende Phosgenierung hergestellt werden (rohes MDI), 4,4-Diphenylmethandiisocya-nat (MDI), 3,3'-Dimethyl-4,4'-diisocyanatobiphenyl und 1,5-Naphthylendiisocyanat. Besonders bevorzugt sind 1,5-Naphthylendiisocyanat und 4,4'-Diphenylmethandiisocyanat.

Als Ausgangskomponenten mit mindestens zwei gegenüber Isocyanaten reaktionsfähigen Wasserstoffatomen von einer Molmasse in der Regel von 400 bis 10 000 und mindestens zwei Zerewitinoff-aktiven Wasserstoffatomen versteht man neben Aminogruppen, Thiolgruppen oder Carboxylgruppen aufweisenden Verbindungen vorzugsweise Hydroxylgruppen aufweisende Verbindungen, insbesondere zwei bis acht Hydroxylgruppen aufweisende Verbindungen, speziell solche der Molmasse 400 bis 10 000, vorzugsweise 1000 bis 6000. Dies sind z.B. mindestens zwei, in der Regel zwei bis drei, Hydroxylgruppen aufweisende Polyester, Polyacetone, Polyether, Polythioether, Polyacetale, Polycarbonate und Polyesteramide, wie sie für die Herstellung von homogenen und von zellförmigen Polyurethanen an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich drei- und vierwertigen Alkoholen mit mehrwertigen, vorzugsweise zweiwertigen Carbonsäuren. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein. Als Beispiele für solche Polycarbonsäuren und deren Derivate seien genannt: Adipinsäure, Sebacinsäure, Phthalsäure, Phthalsäureanhydrid, Tetrahydro- oder Hexahydrophthalsäureanhydrid, Isophthalsäure, Trimellitsäure, Maleinsäureanhydrid, di- und trimerisierte ungesättigte Eettsäuren, Terephthalsäuredimethylester und Terephthalsäure-bis-2-Hydroxyethylester.

Als mehrwertige Alkohole kommen z.B. Ethylenglykol, Propylenglykol, Butandiol-1,4 und/oder -2,3, Hexandiol-1,6, Neopentylglykol, 1,4-Bis-hydroxymethyl-cyclohexan, Hydrochinon-bis-(hydroxyethyl-ether), 2-Methyl-1,3-propandiol, Glycerin, Trimethylolpropan, Hexantriol-1,2,6, Pentaerythrit, Chinit, Mannit und Sorbit, Formit, Methylglykosid, ferner Di-, Tri-, Tetra- und höhere Poly-ethylen-, Poly-propylen-, sowie Poly-butylen-glykole in Frage.

Die Polyester können anteilig endständige Carboxylgruppen aufweisen. Auch Polyester aus Lactonen, z.B. ε-Caprolacton oder Hydroxycarbonsäuren, z.B. ε-Hydroxycapronsäure, sind einsetzbar.

Auch die erfindungsgemäß in Frage kommenden, mindestens 2, in der Regel 2 bis 8, vorzugsweise 2 bis 3,

Hydroxylgruppen aufweisenden Polyether sind solche der an sich bekannten Art und werden z.B. durch Polymerisation von Tetrahydrofuran und/oder Epoxiden wie Ethylenoxid, Propylenoxid, Butylenoxid, Styroloxid oder Epichlorhydrin mit sich selbst, z.B. in Gegenwart von Lewis-Katalysatoren, oder durch Anlagerung dieser Epoxide, vorzugsweise Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z.B. Ethylenglykol, Propylenglykol, Diethylenglykol, Dimethylolpropan, Glycerin, Sorbit, Sucrose, Formit oder Formose, sowie 4,4'-Dihydroxy-di-phenylpropan, Anilin, Ethylendiamin oder Ethanolamin hergestellt. Auch OH-Gruppen aufweisende Polythioether, Polybutadiene, Polyacetale, Polycarbonate oder Polyesteramide sind einsetzbare Ausgangsprodukte.

Vertreter der genannten, erfindungsgemäß zu verwendenden höhermolekularen Polyhydroxylverbindungen sind z.B. in High Polymers, Vol. XVI, "Polyurethanes, Chemistry and Technology", verfaßt von Saunders-Erisch, Interscience Publishers, New York, London, Band I, 1962, Seiten 32 bis 42 und Seiten 44 bis 54 und Band II, 1964, Seiten 5 bis 6 und 198 bis 199, ferner im Kunststoff-Handbuch Band VII, Vieweg-Höchtlen, Carl-Hanser-Verlag, München 1966, z.B. auf den Seiten 45 bis 71, sowie besonders in der DE-OS 2 920 501, Seite 17 bis 24 aufgeführt. Selbstverständlich können Mischungen der o.g. Verbindungen, z.B. Mischungen von Polyethern und Polyestern, eingesetzt werden.

Erfindungsgemäß werden als Kettenverlängerungsmittel c) flüssige Isomerengemische aus der Reihe der 1,4-3,6-Dianhydro-hexitole der allgemeinen Formel (I)

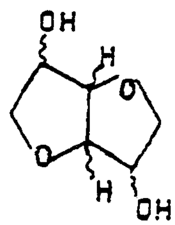 (I)

eingesetzt.

Bevorzugt werden Gemische mit überwiegenden Anteilen an 1,4-3,6-Dianhydro-D-sorbit (Formel (II)) und 1,4-3,6-Dianhydro-D-mannit (Formel III) neben Dianhydroidit (IV).

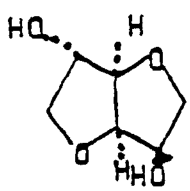
(II)

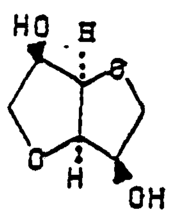
(III)

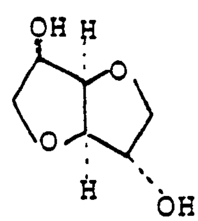
(IV)

Die erfindungsgemäßen Kettenverlängerer-Isomerengemische können auch in Form von weiteren Abmischungen mit weiteren 1,4-3,6-Dianhydro-hexitolen verwendet werden.

Erfindungsgemäß werden auch Mischungen der Bisanhydrohexitol-Isomeren mit anderen kurzkettigen Verbindungen der Molmasse 32 bis 399, vorzugsweise Diolen der Molmasse 62 bis 250, (Kettenverlängerer d)) verwendet.

Als Beispiele für derartige Hydroxylgruppen und/oder Aminogruppen und/oder Thiolgruppen und/oder Carboxylgruppen, vorzugsweise Hydroxylgruppen und/oder Aminogruppen enthaltende Kettenverlängerer d), seien genannt: Niedermolekulare Polyole und/oder Aminoalkohole der Molmasse 62 bis 399, wie Ethylenglykol, Propandiol-(1,2) und -(1,3), Butandiol-(1,4) und -(2,3), Pentandiol-(1,5), Hexandiol-(1,6), Octandiol-(1,8), Neopentylglykol, 1,4-Bis-hydroxymethyl-cyclo-hexan, 2-Methyl-1,3-propandiol, Trimethylolpropan, Hexantriol-(1,2,6), Trimethylolethan, Pentaerythrit, Chinit, Mannit und Sorbit, Ricinusöl, Di- und Tetraethylendiol, höhere Polyethylenglykole, Di-, Tri- und höhere Polypropylendiole, Di-, Tri- und höhere Polybutylendiole mit einer Molmasse bis 399, vorzugsweise bis 250, 4,4'-Dihydroxy-diphenylpropan, Di-hydroxyethylhydrochinon, Terephthalsäure-bis-(2-hydroxyethyl)-ester, Ethanolamin, Diethanolamin und N-Methyldiethanolamin.

Als Beispiele für aromatische Diamine seien Bisanthranilsäureester gemäß den DE-Offenlegungsschriften 2 040 644 und 2 160 590, 3,5- und 2,4-Diaminobenzoe-säureester gemäß DE-A-2 025 900, estergruppenhaltige Diamine, Ethergruppen aufweisende Diamine, gegebenenfalls in 5-Stellung substituierte 2-Halogen-1,3-phenylendiamine, 3,3'-Dichlor-4,4'-diamino-diphenylmethan, Toluylendiamine, 4,4'-Diamino-diphenylmethan, 4,4'-Diaminodiphenylsulfid, Diaminodiphenyldithioether, durch Alkylthiogruppen substituierte aromatische Diamine, Diaminobenzolphosphonsäureester, Sulfonat- oder Carboxylatgruppen enthaltende aromatische Diamine sowie die in der DE-A-2 635 400 aufgeführten hochschmelzenden Diamine genannt. Beispiele für aliphatisch-aromatische Diamine sind Aminoalkylthioaniline.

Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag München 1966, z.B. auf den Seiten 103 bis 113, sowie in der DE-A-2 920 501 beschrieben.

Das erfindungsgemäße Verfahren wird im allgemeinen wie folgt durchgeführt:

Die Reaktionskomponenten werden erfindungsgemäß nach dem an sich bekannten Einstufenverfahren oder dem Prepolymerverfahren zur Umsetzung gebracht, wobei man sich oft maschineller Einrichtungen bedient. Einzelheiten über Verarbeitungseinrichtungen, die auch erfindungsgemäß in Frage kommen, werden im Kunststoff-Handbuch, Band VII, herausgegeben von Vieweg und Höchtlen, Carl-Hanser-Verlag, München 1966, z.B. auf den Seiten 121 bis 247 beschrieben.

Die Mengen an Reaktionskomponenten werden im erfindungsgemäßen Verfahren in der Regel so gewählt, daß das Molverhältnis von Polyisocyanaten zu Kettenverlängerer plus Verbindungen mit reaktionsfähigen OH-Gruppen - abhängig vom jeweils angewendeten Verarbeitungsverfahren - in der Regel zwischen 0,9 und 1,3 liegt, vorzugsweise zwischen 0,95 und 1,25. Der Prozentgehalt an NCO im Prepolymer, falls über die Prepolymerstufe gearbeitet wird, kann 1 bis 18, vorzugsweise 3 bis 16 Gew.-% betragen. Das Molverhältnis von reaktionsfähigem Wasserstoff des Kettenverlängerers zu höhermolekularen Polyhydroxylverbindungen kann in weiten Grenzen variieren, vorzugsweise soll es zwischen 0,5 und 15 liegen, wobei welche bis harte Polyurethan-Typen resultieren.

Eine weitere Ausführungsform besteht darin, daß man die höhermolekulare Verbindung mit mindestens 2 Hydroxylgruppen im Gemisch mit dem Kettenverlängerungsmittel in einem Überschuß an Diisocyanat umsetzt und das Reaktionsprodukt nach der Granulierung in der Hitze unter Druck verformt. Je nach den angewendeten Mengenverhältnissen der Reaktionsteilnehmer können hierbei Polyurethankunststoffe mit verschiedenartigen Härten und verschiedenartiger Elastizität erhalten werden. Man kann auf diese Weise auch Kunststoffe herstellen, die sich wie Thermoplaste verarbeiten lassen. Eine weitere Ausführungsform besteht darin, daß man die höhermolekulare Verbindung mit mindestens 2 Hydroxylgruppen im Gemisch mit dem erfindungsgemäß zu verwendenden Kettenverlängerungsmittel mit einem Unterschuß an Diisocyanat umsetzt, wobei ein walzbares Fell erhalten wird, das anschließend, z.B. durch Vernetzung mit weiterem Diisocyanat, in einen kautschukelastischen Polyurethankunststoff übergeführt werden kann.

Besonders geeignet sind die erfindungsgemäß hergestellten Dianhydrohexitol-Gemische oder ihre Abmischungen mit Diolen beim Elastomer-Gießverfahren mit NCO-Prepolymeren (auf Basis von im wesentlichen linearen Polyestern, Polylaktonen und/oder Polycarbonaten (Molmasse 1000 bis 4000) und überschüssigen Mengen Naphthylen-1,5-diiso-cyanat mit einem NCO-Gehalt von 3 bis 16 %.

Erfindungsgemäß hergestellte Elastomere finden vielseitige Anwendung, z.B. für mechanisch stark beanspruchte Formkörper, wie Rollen, Keilriemen oder Dichtungen, die thermisch oder chemisch stark beansprucht werden, für Heißwasserrohre oder Motoren oder zur Herstellung von Folien, Textilbeschichtungen und Polyurethanpulvern. Neben homogenen Elastomeren können auch zellförmige Polyurethane (weiche bis harte Schaumstoffe) hergestellt werden.

Die folgenden Beispiele erläutern das erfindungsgemäße Verfahren. Wo nicht anders vermerkt, sind Zahlenwerte als Gewichtsteile bzw. Gewichtsprozente zu verstehen.

### (A) Acylierung von Sorbit (statistisch acyliertes "Di"acetat)

#### Beispiel 1

1 Mol Sorbit (182 g) und 170 g Essigsäure werden mit 2 g konzentrierter Schwefelsäure versetzt und auf ca. 105°C Innentemperatur gebracht, wobei Wasser über eine Kolonne abdestilliert wird. Nach beendeter Wasserdestillation wird mit halbkonzentrierter Natronlauge neutralgestellt und die überschüssige Essigsäure und Wasser abdestilliert, wobei Vakuum zweckmäßig ist. Zur restlosen Wasser-/Essigsäure-Beseitigung ist eine 3-malige Destillation mit Toluol (3 x 200 ml) effektiv.

Alternativ kann das statistisch acylierte Diacetat im Vakuum (0,013 mbar) bei 180 bis 240° C destilliert werden.

**Beispiel 2**

1 Mol Sorbit (182 g) und 2 Mol Essigsäureanhydrid (204g) werden zusammen auf ca. 120 bis 130° C Innentemperatur erwärmt und an einem gut wirksamen Rückflußkühler 2 Stunden am Rückfluß gekocht, bis die Rückflußtemperatur 118° C erreicht hat. Abdestillieren der Essigsäure, erfolgt gemäß Beispiel 1.

Die Reaktion kann wahlweise auch mit Natriumacetat oder Säure katalysiert werden.

**Beispiel 3**

Zu 1 Mol Sorbit (182 g) werden bei Raumtemperatur 2 Mol Acetylchlorid gegeben und erwärmt, wobei Chlorwasserstoff entweicht. Anfangs soll Acetylchlorid am Rückfluß kochen, dann kann die Innentemperatur auf ca. 100° C erhöht werden.

Neutralisation kann z.B. Mit Natriumacetat erfolgen, wobei wie unter Beispiel 1 aufgearbeitet wird. Das zurückbleibende Natriumchlorid stört im weiteren Arbeitsgangs nicht.

Wird Sorbit oder Mannit in den Beispielen 1-3 als wäßrige Lösung eingesetzt, muß vorher das Wasser (gegebenenfalls im Vakuum) abdestilliert werden.

**(B) Dianhydrohexitol-Diacetate**

**Beispiel 4**

2 Mol Hexit-Diacetat werden mit 3 g konzentrierter Schwefelsäure innerhalb von ca. 1 bis 1 1/4 h auf eine Heizbad-Temperatur von 180 bis 190° C gebracht, wobei gegebenenfalls im Vakuum Wasser abdestilliert wird. Nach 1 1/4 h wird eine 2,2 g Natriumhydroxid enthaltende 30 %ige wäßrige Lösung vorsichtig in das Reaktionsgemisch getropft und fraktioniert. Bei 170 bis 200° C/26 mbar geht ein blaßgelbes Öl des Dianhydrohexit-Diacetats über, wobei außer Salz annähernd kein Rückstand bleibt.

Anstelle von Schwefelsäure kann ebenfalls p-Toluolsulfonsäure, Chlorwasserstoff, Bortrifluorid-Etherat etc. verwendet werden.

**(C) Verseifung des Acetatgemisches**

**Beispiel 5**

Das Destillat aus Beispiel 4 wird in Ethanol gelöst, so daß eine 60 %ige Lösung entsteht, und anschließend wird mit Natriummethylat auf pH = 9-10 gestellt. Nach 5 Stunden bei Raumtemperatur wird mit verdünnter Salzsäure neutralisiert und destilliert. Man erhält, bezogen auf 2 Mol Sorbit nach Beispielen 1-3, 232 g blaßgelbes Dianhydrohexitolgemisch (ca. 80 % der Theorie). Nach GC sind etwa 7 bis 12 % Dianhydromannit, 79 bis 83 % Dianhydrosorbit, 6 bis 9 % Dianhydroidit und bis etwa 4 % von mono-, di- und trihydroxyfunktionellen Verunreinigungen enthalten.

Die Verseifung mit wäßriger Natronlauge oder mit anderen üblichen Basen liefert naturgemäß gleiche Ergebnisse.

Das Verfahren kann selbstverständlich auch ohne Isolierung der einzelnen Stufen durchgeführt werden. Dies wird durch das folgende Beispiel illustriert:

**Beispiel 6**

730 g einer 50 %igen wäßrigen Lösung von 70 Teilen Sorbit und 30 Teilen Mannit werden im Vakuum (20 mm) bei 100° C zu einem Öl eingeengt, zu dem nach beendeter serdestillation 180 g Acetanhydrid und 6,5 g p-Toluolsulfonsäure. $H_2O$ gegeben werden.

Es wird auf 130 bis 140° C erwärmt und wieder Wasser und Essigsäure abdestilliert. Innerhalb von 1 bis 1,5 h wird auf 180 bis 190° C (Heizbad-Temperatur) aufgeheizt, während das Wasser aus der Ringschluß-Reaktion abdestilliert.

Nach beendeter Wasserabspaltung wird auf ca. 100 bis 130° C gekühlt und eine Lösung von 3,76 g

Natriummethylat in 40 ml n-Butanol zugetropft. Die basische Mischung wird ca. 20 Minuten bei 130°C gehalten und anschließend zur Neutralisation nochmals 6,5 g p-Toluolsulfonsäure. $H_2O$ zugegeben. Nach Fraktionierung im Vakuum erhält man 232 g blaßgelbes Dianhydrohexitolgemisch (80 % der Theorie).

Es enthält nach GC 29 bis 33 % Dianhydromannit, 57 bis 62,% Dianhydrosorbit und 6 bis 9 % Dianhydroidit sowie etwa bis 4 % mono-, di- und trihydroxyfunktioneller Verunreinigungen.

**Beispiel 7**

2 Mol Sorbit (364 g) werden bei 130 bis 140°C geschmolzen und zur Schmelze 2 Mol Acetanhydrid (204 g) zugetropft, wobei die bei der Acylierung gebildete Essigsäure am Rückfluß kocht.

Nach ca. 15 Minuten werden 3 g konzentrierte Schwefelsäure zugegeben. Während die Temperatur des Heizbades innerhalb von 1 bis 1,5 h auf 180 bis 190°C erhöht wird, bildet sich spontan Wasser, das abdestilliert wird.

Nach 1,5 h wird eine 2,2 g NaOH enthaltende, 30 %ige wäßrige Lösung zur Neutralisation vorsichtig in das Gemisch getropft und fraktioniert destilliert. Es geht ein blaßgelbes Öl des Diacetyl-dianhydrohexitol-Gemisches bei 170 bis 200°C/26 mbar über, wobei außer Salz praktisch kein Rückstand bleibt.

Das Öl wird in 300 ml Methanol mit soviel Natriummethylat versetzt, bis pH = 10 erreicht ist und wird weiterhin am Rückfluß gekocht. Nach beendeter Entacetylierung wird mit Schwefelsäure neutralisiert und fraktioniert destilliert. Man erhält einen Vorlauf aus Essigester und Alkohol, dann destilliert das blaßgelbe Produkt des Dianhydrohexitol-Isomerengemischs (ein Öl) bei 170 bis 190°C/26 mbar über. Ausbeute: 252 g = 86 % der Theorie.

Das Öl aus Dianhydrosorbit, Dianhydromannit und Dianhydroidit bleibt über Monate flüssig.

**Beispiel 8**

250 Teile eines aus Adipinsäure und Ethylenglykol hergestellten Polyesters mit einer mittleren Molmasse von 2000 werden bei 120°C und einem Vakuum von 14 mbar entwässert.

Dann gibt man 44,6 Teile 1,5-Diisocyanatonaphthalin zu und hält die Temperatur 30 Minuten bei 130°C. Zu dem NCO-Prepolymer wird nur unter starkem Rühren bei 110°C eine äquivalente Menge (NCO/OH= 1:1) flüssiges Isomerengemisch nach Beispiel 7 und 0,035 Teile, Zinn-(II)-octoat gegeben. Es wird 30 Sekunden gerührt und dann in eine auf 110°C vorgeheizte Form gegossen. Die Mischung ist 3-4 Minuten gießfähig. Es wird noch 24 Stunden bei 110°C getempert. Das fertige, homogene Elastomer hat folgende Eigenschaften:

| | |
|---|---|
| Zugfestigkeit: | 32,3 MPa |
| Reißdehnung: | 650 % |
| Strukturfestigkeit | 500 N |
| Shore-Härte A: | 75 |
| Elastizität: | 41 %. |

**Beispiel 9**

200 Teile eines Mischpolyesters aus Adipinsäure, Ethylenglykol, 1,4-Butandiol (1:1) (Molmasse 2000) werden 30 Minuten bei 13 mbar und 130°C entwässert. Dann gibt man 80 Teile 1,5-Diisocyanatonaphthalin zu. Man hält 30 Minuten bei 130°C und kühlt dann auf 110°C. Es wird eine Mischung aus 34 Teilen Dianhydrohexitolen nach Beispiel 7 und 0,067 Teilen Triethylendiamin (DABCO ®) eingerührt. Nach 30 Sekunden wird vergossen. Die Mischung ist 2 Minuten gießfähig. Das 24 Stunden bei 110°C getemperte Produkt hat folgendes Werteprofil:

| | |
|---|---|
| Zugfestigkeit: | 22,8 MPa |
| Reißdehnung: | 310 % |
| Strukturfestigkeit | 630 N |
| Shore-Härte A: | 96 |
| Elastizität: | 39 %. |

**Beispiel 10**

Man stellt eine flüssige, kristallisationsstabile Mischung aus 91,7 Teilen 1,4-3,6-Dianhydro:D-hexiten nach beispiel 7 und 9,1 Teilen 1,4-Butandiol her. Zu 32,2 Teilen der so hergestellten Mischung werden 0,66 Teile DABCO ® gegeben. Dieses Gemisch wird mit dem Prepolymer aus Beispiel 9 verrührt. Die Mischung ist 6 Minuten gießfähig. Das fertige Elastomer hat folgende Kenndaten:

Zugfestigkeit: 22,5 MPa
Reißdehnung: 310 %
Strukturfestigkeit 590 N
Shore-Härte A: 93
Elastizität: 37 %.

**Patentansprüche**

1. Verfahren zur Herstellung von flüssigen 1,4-3,6-Dianhydrohexitol-Gemisch durch Wasserabspaltung aus Hexitolen in Gegenwart von starken Säuren und Acylierungsmitteln bei Temperaturen ⩾ 130° C dadurch gekennzeichnet, daß man

Sorbit und/oder Mannit, vorzugsweise in Abwesenheit von Wasser oder einem zusätzlichen Lösungsmittel, mit 1,5-2,5 Äquivalenten an unter den Reaktionsbedingungen acylierend wirkenden Substanzen aus der Reihe der Carbonsäuren oder ihrer Anhydride, Halogenide oder Kohlensäureesterderivate sowie Keten pro Mol Hexitol in die Diacylierungsprodukte überführt, eventuell überschüssiges Acylierungsmittel, evtl. gebildetes Wasser, HCl oder Carbonsäuren entfernt,

die Diacylierungsprodukte der Hexitole in Gegenwart von 0,001 bis 10 Mol-% löslichen Proton- oder Lewissäuren oder heterogenen, sauren Katalysatoren bei Temperaturen von 170-240° C unter Wasserabspaltung und Isomerisierung zu den im wesentlichen diacylierten Dianhydrohexitol-Isomerengemischen umsetzt

und das acylierte Dianhydrohexitol-Isomerengemisch, gegebenenfalls nach der Destillation, durch Verseifung oder Umesterung in ein flüssiges 1,4-3,6-Dianhydrohexi-tol-Isomerengemisch von geringer Kristallisationsneigung und mit einem Gehalt an 0,5-12 Gew.-% an 1,4-3,6-Dian-hydroidit-Isomeren überführt.

2. Verfahren nach Anspruch 1, dadurch gekennzeichnet, daß man als Acylierungsmittel Essigsäure, Acetanhydrid oder Acetylchlorid verwendet.

3. Verfahren nach Ansprüchen 1 und 2, dadurch gekennzeichnet, daß man die Di-acylierung in Gegenwart von löslichen Proton- oder Lewis-Säuren bei 175 bis 210° C unter Wasserabspaltung durchführt.

4. Verfahren nach Ansprüchen 1 bis 3, dadurch gekennzeichnet, daß man die Überführung der Hexite in die Dianhydrohexitol-Isomerengemische ohne Isolierung der Zwischenstufen durchführt.

5. Verfahren nach Ansprüchen 1 bis 4, dadurch gekennzeichnet, daß man die Isomerisierungsreaktion mit Sorbit durchführt.

6. Verfahren nach Ansprüchen 1 bis 4, dadurch gekennzeichnet, daß man die Isomerisierungsreaktion mit 95:5 bis 5:95-Gemischen von Sorbit und Mannit durchführt.

7. Flüssiges 1,4-3,6-Dianhydrohexitol-Isomerengemisch von geringer Kristallisationsneigung und mit einem Gehalt an 0,5-12 Gew.-% an 1,4-3,6-Dianhydro-idit-Isomeren, dadurch gekennzeichnet, daß man sie nach dem Verfahren der Ansprüche 1-6 herstellt.

8. Flüssiges 1,4-3,6-Dianhydrohexitol-Isomerengemisch nach Anspruch 7, dadurch gekennzeichnet, daß es einen Gehalt von 2-9 Gew.-% 1,4-3,6-Dianhydroidit neben 1,4-3,6 Dianhydrosorbit und 1,4-3,6-Dianhydromannit aufweist.

9. Verwendung von Dianhydrohexitolen in einem Verfahren zur Herstellung von homogenen und zellförmigen Polyurethankunststoffen durch Umsetzung von

a) organischen Polyisocyanaten mit

b) mindestens 2 Zerewitinoff-aktiven Wasserstoffatome aufweisenden Verbindungen der Molmasse 400 bis 10 000;

c) Kettenverlängerungsmitteln,

d) gegebenenfalls weiteren, mindestens 2 Zerwitinoff-aktive Wasserstoffatome aufweisenden kurzkettigen Verbindungen der Molmasse 32 bis 399,

e) sowie gegebenenfalls Katalysatoren, Treibmitteln und weiteren an sich bekannten Zusatzstoffen,

dadurch gekennzeichnet, daß man als Kettenverlängerer c) die nach den Verfahren der Ansprüche 1-6 hergestellten flüssigen Isomerengemische der 1,4-3,6-Dianhydrohexitole, gemäß den Ansprüchen 7 oder 8, gegebenenfalls in Abmischung mit niedermolekularen Verbindungen d) der Molmasse 62-339, einsetzt.

10. Verwendung nach Anspruch 9, dadurch gekennzeichnet, daß man im Elastomer-Gießverfahren NCO-Prepolymere mit einem NCO-Gehalt von 3 bis 16 Gew.-% auf Basis von im wesentlichen linearen Polyestern, Polylactonen und/oder Polycarbonaten der Molmasse 1000 bis 4000 und überschüssigen Mengen an Naphthylen-1,5-diisocyanat, mit den flüssigen Dianhydro-hexitol-Isomerengemischen gemäß den Ansprüchen 7 oder 8 oder ihren Abmischungen mit Diolen, umsetzt.

**Claims**

1. Process for the production of liquid 1,4-3,6-dianhydro-hexitol mixtures by the elimination of water from hexitols in the presence of strong acids and acylating agents at temperatures of ⩾ 130° C, characterised in that

sorbitol and/or mannitol are converted, preferably in the absence of water or an additional solvent, into the diacylation products, with 1.5-2.5 equivalents of substances having an acylating action under the reaction conditions, from the series comprising the carboxylic acids or their anhydrides, halides or carbonic acid ester derivatives and ketene, per mol of hexitol, any excess acylating agent, any water formed, HCl or carboxylic acids are removed, the diacylation products of the hexitols are reacted, in the presence of 0.001 to 10 mol % of soluble proton or Lewis acids or heterogeneous, acid catalysts, at temperatures of 170-240°C, with the elimination of water and isomerisation to form the substantially diacylated dianhydrohexitol isomer mixtures and the acylated dianhydrohexitol isomer mixture is converted, optionally after distillation, by saponification or transesterification, into a liquid 1,4-3,6-dianhydrohexitol isomer mixture having very little tendency towards crystallisation and a content of 0.5-12% by weight of 1,4-3,6-dianhydroiditol isomers.

2. Process according to Claim 1, characterised in that acetic acid, acetic anhydride or acetyl chloride are used as the acylating agents.

3. Process according to Claims 1 and 2, characterised in that the diacylation is carried out in the presence of soluble proton or Lewis acids at 175 to 210°C with the elimination of water.

4. Process according to Claims 1 to 3, characterised in that the conversion of the hexitols into the dianhydrohexitol isomer mixtures is carried out without isolation of the intermediate stages.

5. Process according to Claims 1 to 4, characterised in that the isomerisation reaction is carried out with sorbitol.

6. Process according to Claims 1 to 4, characterised in that the isomerisation reaction is carried out with 95:5 to 5:95 mixtures of sorbitol and mannitol.

7. Liquid 1,4-3,6-dianhydrohexitol isomer mixture having very little tendency towards crystallisation and having a content of 0.5-12% by weight of 1,4-3,6-dianhydroiditol isomers, characterised in that they are produced by the process of Claims 1-6.

8. Liquid 1,4-3,6-dianhydrohexitol isomer mixture according to Claim 7, characterised in that it has a content of 2-9% by weight of 1,4-3,6-dianhydroiditol in addition to 1,4-3,6-dianhydrosorbitol and 1,4-3,6-dianhydromannitol.

9. Use of dianhydrohexitols in a process for the production of homogeneous and cellular polyurethane plastics by reacting

a) organic polyisocyanates with

b) compounds containing at least 2 Zerewitinoff-active hydrogen atoms and having a molecular weight of 400 to 10,000;

c) chain-extending agents,

d) optionally other short-chain compounds containing at least 2 Zerewitinoff-active hydrogen atoms and having a molecular weight of 32 to 399,

e) and optionally catalysts, blowing agents and other additives known per se,

characterised in that the chain-extending agents c) used are the liquid isomer mixtures of 1,4-3,6-dianhydrohexitols, according to Claims 7 or 8, produced according to the processes of Claims 1-6, optionally mixed with low molecular weight compounds d) having a molecular weight of 62-339.

10. Use according to Claim 9, characterised in that NCO prepolymers having an NCO content of 3 to 16% by weight and based on substantially linear polyesters, polylactones and/or polycarbonates of a molecular weight of 1,000 to 4,000 and excess quantities of naphthylene-1,5-diisocyanate, are reacted with the liquid dianhydrohexitol isomer mixtures according to Claims 7 or 8, or mixtures thereof with diols, by the elastomer casting process.

## Revendications

1. Procédé de fabrication de mélanges liquides de 1,4-3,6-dianhydrohexitols par clivage de l'eau à partir d'hexitols en présence d'acides forts et d'agents d'acylation à des températures égales ou supérieures à 130°C,

caractérisé en ce qu'on convertit en produits de diacylation du sorbitol et/ou du mannitol, de préférence en l'absence d'eau ou d'un solvant supplémentaire, avec 1,5-2,5 équivalents par mole d'hexitol de substances à action acylante dans les conditions de la réaction et qui appartiennent à la série des acides carboxyliques ou de leurs anhydrides, halogénures, des dérivés esters d'acide carbonique de même que du cétène, on élimine éventuellement l'agent d'acylation en excès, éventuellement l'eau formée, le HCl ou les acides carboxyliques, on fait réagir les produits de diacylation des hexitols en présence de 0,001 à 10 moles % d'acides protoniques ou de Lewis solubles ou de catalyseurs acides hétérogènes à des températures de 170 - 240°C avec clivage d'eau et isomérisation pour obtenir ainsi les mélanges isomères de dianhydrohexitols essentiellement diacylés et l'on convertit le mélange d'isomères de dianhydrohexitols acylés, éventuellement après la distillation, par saponification ou transestérification en un mélange liquide d'isomères de 1,4-3,6-dianhydrohexitols à faible tendance à la cristallisation et comportant une teneur de 0,5 à 12% en poids en isomères de 1,4-3,6-dianhydroiditol.

2. Procédé selon la revendication 1, caractérisé en ce qu'on utilise comme agent d'acylation de l'acide acétique, de l'anhydride acétique ou du chlorure d'acétyle.

3. Procédé selon les revendications 1 et 2, caractérisé en ce qu'on exécute la diacylation en présence d'acides protoniques ou de Lewis solubles à 175 - 210° C avec clivage d'eau.

4. Procédé selon les revendications 1 à 3, caractérisé en ce qu'on exécute la conversion des hexitols en mélanges isomères de dianhydrohexitols sans isolation des stades intermédiaires.

5. Procédé selon les revendications 1 à 4, caractérisé en ce qu'on effectue la réaction d'isomérisation avec du sorbitol.

6. Procédé selon les revendications 1 a 4, caractérisé en ce qu'on effectue la réaction d'isomérisation avec des mélanges 95:5 à 5:95 de sorbitol et de mannitol.

7. Mélange liquide d'isomères de 1,4-3,6-dianhydrohexitols à faible tendance à la cristallisation et ayant une teneur de 0,5 à 12% en poids en isomères de 1,4-3,6-dianhydroiditol, caractérisé en ce qu'on le prépare par le procédé des revendications 1 à 6.

8. Mélange liquide d'isomères de 1,4-3,6-dianhydrohexitols selon la revendication 7, caractérisé en ce qu'il présente une teneur de 2 à 9% en poids de 1,4-3,6-dianhydroiditol à côté de 1,4-3,6-dianhydrosorbitol et de 1,4-3,6-dianhydro-mannitol.

9. Utilisation de dianhydrohexitols dans un procédé de fabrication de matières plastiques homogènes et cellulaires en polyuréthanes par réaction

a) de polyisocyanates organiques avec

b) des composés de masse molaire de 400 à 10 000 présentant au moins 2 atomes d'hydrogène actifs selon Zéréwitinoff,

c) des agents d'allongement de chaîne,

d) éventuellement d'autres composés de masse molaire de 32 à 399 à chaîne courte présentant au moins 2 atomes d'hydrogène actifs selon Zéréwitinoff

e) de même qu'éventuellement des catalyseurs, des agents expanseurs et d'autres additifs connus en eux-mêmes,

caractérisée en ce qu'on emploie comme agent d'allongement de chaîne c) les mélanges liquides d'isomères des 1,4-3,6-dianhydrohexitols préparés par les procédés des revendications 1 à 6, selon les revendications 7 ou 8, éventuellement en coupage avec des composés à poids moléculaire inférieur d) de masse molaire de 62 à 339.

10. Utilisation selon la revendication 9, caractérisée en ce que dans le procédé de coulée d'élastomères on fait réagir des prépolymères à NCO ayant une teneur en NCO de 3 à 16% en poids, à base de polyesters, polylactones et/ou polycarbonates essentiellement linéaires et d'une masse molaire de 1000 à 4000 et de quantités excédentaires de naphtylène-1,5-diisocyanate, avec les mélanges liquides d'isomères selon les revendications 7 ou 8 de dianhydrohexitols ou de leurs mélanges avec des diols.